# EUROPEAN PATENT APPLICATION

(11) **EP 4 407 021 A1**
(43) Date of publication of application: **31.07.2024**
(21) Application number: 23153703.6
(22) Date of filing: 27.01.2023
(51) Int. Cl.: C12M 1/32, C12M 3/06, C12M 1/00

(54) **BIOCHIP FOR CELL TRANSPORT**

(71) Applicant: TECHNISCHE UNIVERSITÄT WIEN, 1040 Wien (AT); Medizinische Universität Graz, 8010 Graz (AT)
(72) Inventor: Selinger, Florian, 1040 Wien (AT); Ertl, Peter, 1040 Wien (AT); Rinner, Beate, 8045 Weinitzen (AT)
(74) Representative: Schwarz & Partner Patentanwälte GmbH

(57) **Abstract**

The present invention relates to a microfluidic device (1;10) for the storage and/or transport of cells, in particular cell aggregates, wherein the device (1;10) comprises at least one chamber (2) comprising at least one opening (3) for introducing a fluid into the chamber (2) and at least one opening (4) for removing a fluid from the chamber (2), wherein the at least one chamber (2) comprises a base (5;11) and a top (6;12), wherein the top (6;12) is situated on the opposite to the base (5;11), wherein the base (5;11) is formed by a substrate comprising at least one recess (7) for collecting a fluid, wherein the distance (8) between the base (5;11) and the top (6;12) of the at least one chamber (2) is less than the width (9) of the at least one recess (7), and wherein the width (9) of the at least one recess (7) is the smallest line segment between two opposite points passing through the center of the base area of the at least one recess (7).

## Description

### FIELD OF THE INVENTION

The present invention relates to a microfluidic device for the storage and/or transport of cells.

### BACKGROUND ART

Handling, cultivation, and analysis of living cell cultures are commonly utilized methods in biomedical research and development as well as in pharmaceutical industry. Primary cell culture and continuous cell lines are widely used for the cultivation of viruses, the production of biopharmaceutical substances, efficacy studies, and toxicity tests. Hence, cell cultures are globally used by scientists who require a broad variety of cells depending on research topic. To meet the growing demand for living cell cultures, cells are shipped around the globe from cell banks to research and production facilities. However, the transport of living cells is challenging since viability of the cells has to be maintained during shipment. Another important aspect of cell transport relates to high costs. For instance, Germany's second largest cell bank (Cell Line Service; CLS) spends between 40.000 and 50.000 euros each year for cell shipment. The costs mainly depend on the type of shipment and customs regulation of the recipient country (e.g., EU, non-EU country).

Currently two shipment options are commonly used, including the transport of viable cells or the transport of frozen cells on dry ice.

When shipping viable cells, cells are transported under thawed conditions in cell culture flasks. Different flask sizes can be chosen depending on cell type and shipping time. Standard flask sizes for shipping are e.g., flasks having 25 cm² or 75 cm² surface area (with respect to the growth surface) with a media volume of approx. 20 to 70 mL. During transport, the cells are viable and have the ability to divide. The conditions during the transportation of viable cells severely differ from optimal cell culture conditions such as adequate temperature at 37°C, optimal CO₂ supply, and a suitable amount of media, resulting in a restricted cell growth behavior during transportation.

Cultured cells can also be shipped using dry ice - the solid form of carbon dioxide - which keeps cells frozen at - 80°C. However, dry ice shipping is expensive, voluminous, and hazardous, so many couriers refuse to handle it. Also, if the dry ice evaporates before a shipment reaches its destination, cellular recovery is jeopardized, as the cells get crushed and the cryoprotectant needed for their storage at low temperatures is toxic to cells at ambient temperatures. Therefore, dry ice must be handled with caution, and defined safety measures have to be conducted. It often turns out that the viability of cells shipped on dry ice is reduced up to 30% upon arrival at the recipient. Different success rates in terms of growth behavior between adherent and suspension cultures at the recipient laboratory can also be observed.

Therefore, it is an object of the present invention to provide means and methods to enhance and facilitate the transport of cells and cell aggregates.

### SUMMARY OF THE INVENTION

Thus, the present invention relates to a microfluidic device for the storage and/or transport of cells, in particular cell aggregates, wherein the device comprises at least one chamber comprising at least one opening for introducing a fluid into the chamber and at least one opening for removing a fluid from the chamber, wherein the at least one chamber comprises a base and a top, wherein the top is situated on the opposite to the base, wherein the base is formed by a substrate comprising at least one recess for collecting a fluid, wherein the distance between the base and the top of the at least one chamber is less than the width of the at least one recess, and wherein the width of the at least one recess is the smallest line segment between two opposite points passing through the center of the base area of the at least one recess.

It turned surprisingly out that the present invention shows exceptional capabilities for the transport and/or storage of cells, in particular as cell aggregates. Cells and cell aggregates may reside safely in at least one recess of the microfluidic device of the present invention. With the microfluidic device according to the invention it is therefore possible to transport and/or store cells and cell aggregates in a reliable, compact, and cost-efficient manner. The transport and/or storage of cells in the form of aggregates is even more beneficial since the aggregated cells only need a limited space for storage and/or transportation due to compact morphology and show enhanced resistance against harmful influences. Furthermore, cell aggregates have typically a size which is larger than the dimensions of the chamber of the microfluidic device, so that the cell aggregate cannot move out of the recess. The cell aggregates reside in one or more recesses of the microfluidic device of the present invention, even at severe vibrations and shocks during storage and/or transport. This is particular advantageous since viable cells are typically sensitive.

The size (as e.g., the diameters) of the cell aggregates can be restricted by the width of the at least one recess. For instance, the size of a cell aggregate is confined by the width of the recess, wherein the height of the chamber is less than the width of the recess. Hence, the cell aggregate is larger than the height of the chamber and cannot flow back into the chamber due to the geometric restrictions.

Additionally, only small amounts of fluids for the storage and/or transport of cell aggregates are required due to the limited volume of the microfluidic device of the present invention. For instance, cell aggregates can be shipped at significant lower costs in comparison to present shipment methods due to limited fluid volumes needed and reduced packaging weight.

Another aspect of the present invention relates to a method for the storage and/or transport of cells, in particular cell aggregates, comprising the steps of:
a) applying a fluid comprising cells into the at least one chamber of the device as defined herein and thus providing said fluid to the at least one recess, and
b) incubating the cells until at least one aggregate is formed in the at least one recess.

It turned out that the cell aggregates can be efficiently stored and/or transported by the method of the present invention. Cell aggregates can be formed by simply adding a fluid comprising individual cells into the at least one chamber, in particular in at least one recess, of the microfluidic device of the present invention. After formation, the cell aggregates are hindered to be washed-out out from the at least one recess due to the lower height of the chamber compared to the width of the recess. This feature has the advantageous effect that a) individual cells can flow through the at least one chamber and settle in the at least one recess after applying a fluid comprising the cells and b) cell aggregates residing in the at least one recess are safely formed.

A further aspect of the present invention relates to the use of the device as defined herein for the storage and/or transport of cells, in particular cell aggregates.

Yet another aspect of the present invention relates to a method for the screening of an effect of compounds on cell aggregates comprising the steps of:
a) providing a solution comprising cells,
b) applying the solution of step a) into the at least one chamber of the device as defined herein and thus providing said fluid to the at least one recess,
c) incubating the cells until at least one cell aggregate is formed in the at least one recess,
d) treating the at least one cell aggregate with at least one compound, and
d) measuring an effect of the at least one compound on said at least one cell aggregate.

With the method of the present invention, cell aggregates can be generated and analyzed in a reliable and reproducible manner on a single device. Further, the microfluidic device may be compatible with standardized laboratory equipment. As example, fluids, cells, compounds, etc. can be applied to the device by microchannel pipettes, pipetting robots, and effects of compounds on cell aggregates can be determined by state-of-the-art plate readers. In particular, the present method is useful in the area of biomedical research, drug screening, and personalized medicine. In particular, the physiological state of a patient can be profiled in a multi-parametric and multimodal manner by the present invention to identify preclinical stages of diseases and to make adequate differential diagnoses.

### SHORT DESCRIPTION OF THE FIGURES

Fig. 1a shows a schematic plan view of an embodiment of a microfluidic device according to the present invention.
Fig. 1b shows a sectional side view of the embodiment of the microfluidic device according to Fig. 1a with hemispherical-shaped recesses and truncated cone-shaped hollow bodies.
Fig. 1c shows a sectional side view of the embodiment of the microfluidic device according to Fig. 1a including the fluid chamber and hemispherical-shaped recesses with truncated cone-shaped hollow bodies.
Fig. 2a shows a schematic plan view of another embodiment of the microfluidic device according to the present invention.
Fig. 2b shows a sectional side view of the embodiment of the microfluidic device according to Fig. 2a including the fluid chamber and a hemispherical-shaped recess and a truncated-cone shaped hollow body.
Fig. 3a shows a schematic plan view of the embodiment of the base of the microfluidic device according to Fig. 1a.
Fig. 3b shows a schematic plan view of the embodiment of the base of the microfluidic device according to Fig. 2a.
Fig. 4a and Fig. 4b show a schematic top view and a schematic side view of the top layer of the embodiment of the microfluidic device according to Fig. 1a.
Fig. 5 shows a schematic workflow of the method according to the present invention for the screening of compounds on cell aggregates.
Fig. 6a shows schematically pictures of the different chamber heights (50 pm, 100 pm, 150 µm) at constant recess widths.
Fig. 6b shows flow profiles of a microfluidic flow in the chamber of the device according to the present invention at different chamber heights.
Fig. 7a shows schematically pictures of different hollow body widths (500 pm, 600 pm, 700 pm, 800 pm, 9000 pm) at a constant width (500 pm) of the recesses.
Fig. 7b shows flow profiles of the microfluidic flow in the chamber of the device according to the present invention at different hollow body widths.
Fig. 8 shows cellular viability of cell aggregates after storage in the microfluidic device according to the present invention overnight at 37°C and at room temperature (RT).
Fig. 9 shows fluorescence micrographs of harvested cells from the microfluidic device according to the invention when seeded in a cell culture plate. The cells were applied at six different densities to the microfluidic device.

### EMBODIMENTS OF THE INVENTION

"Cells", as used herein, refers to membrane enclosed particles that may comprise proteins, nucleic acids, lipids, metabolites and/or organelles. Cells may grow and/or divide into other cells or release other cells. According to another preferred embodiment of the present invention the cells are selected from the group consisting of eukaryotic cells, prokaryotic cells, extracellular vesicles, and combinations thereof. In particular, the eukaryotic cell may be a mammalian cell, preferably an epithelial cell, a nerve cell, a muscle cell, a connective tissue cell, a stem cell, preferably a tumor cell. The prokaryotic cell may be bacterial cell or an archaeal cell. In particular, the extracellular vesicle may be an exo-some, a microvesicle or an apoptotic body. The cells according to the present invention may have a size between 80 nm to 100 pm, preferably between 90 nm to 80 pm, more preferably between 100 nm to 60 µm.

The term "cell aggregate" refers to a three-dimensional micromass of individual cells suspended in a fluid. Cell aggregates according to the present invention are formed by the aggregation of cells. The individual cells can stick, bind, attach or connect to each other to form an aggregate. Induvial cells can also fuse together to form a cell aggregate. The aggregate may have a size between 60 µm and 3 mm, preferably between 80 µm and 2 mm, preferably between 100 µm and 1 mm. Cell aggregation can be a reversible or irreversible process.

The "size" of the cells can be determined by methods known in the art (e.g. electron microscopy).

In the course of aggregation, the cell aggregates may grow in size, and may sediment. The reverse process, whereby cell aggregates are re-dispersed as individual cells, hardly occurs spontaneously but may occur under mechanical and/or chemical disaggregation. In particular, a cell aggregate consisting of eukaryotic cells is a three-dimensional cell micromass in the form of a spheroid or having a spheroid-like form. Such spheroids can also be considered as "organoids". "Cellular spheroids" can be formed by eukaryotic, in particular mammalian cells (e.g., human cells), whereby particularly preferred cells are cells being present in organs and tissues of mammals. These spheroids may comprise one or more type of cells. The use of a different type of cells allows the production of more complex "organoids" or tissue-like structures. A cell aggregate can also be a three-dimensional micromass of extracellular vesicles or a combination of eukaryotic cells or prokaryotic cells and extracellular vesicles having a spheroid-like form.

The "base", as used herein, refers to the inner bottom surface of the at least one chamber and is substantially situated below the fluid introduced into the chamber and is situated parallel to the fluid flow direction. The "top", as used herein, refers to the inner top surface of the at least one chamber and is situated opposite to the inner bottom surface. The "base area", as used herein, refers to the two-dimensional intersection area of a three-dimensional geometric body. Preferably, the base area of the at least one recess has the shape of a circle or an ellipse.

The cells and/or cell aggregates according to the present invention can be stored and/or transported in a fluid. The fluid is introduced via the at least one opening into the at least one chamber of the device of the present invention. Said fluid can also be applied by introducing it into the at least one chamber via at least one fluid reservoir. "Fluid", as used herein, refers to a liquid fluid.

According to another preferred embodiment of the present invention the at least one opening is connected to at least one fluid reservoir. The at least one reservoir has the advantage that it can be filled with sufficient fluid required for the storage and/or the transport of the cell aggregates. The fluid reservoir of the present invention may have any shape and may have a defined volume for taking up, for instance, sufficient fluid to supply the at least one chamber with fluid at a certain level. Depending on the anticipated storage and/or transportation time of said cell aggregates as well as the intended area of use, the volume of the at least one reservoir can be adapted. One major advantage of the present invention is that the fluid volume needed for the transport and/or storage of the cell aggregates is significantly reduced compared to e.g., the volume of a culture flask.

According to another preferred embodiment of the present invention the at least one reservoir has a volume of 1 mm³ to 5000 mm³, preferably 2 mm³ to 4000 mm³, preferably 5 mm³ to 2000 mm³, preferably 10 mm³ to 1000 mm³, preferably 20 mm³ to 500 mm³.

In order to avoid loss and wash-out of cell aggregates during storage and/or transport from the at least one recess of the present invention the distance between the base and the top of the at least one chamber is less than the width of the at least one recess. Due to this geometric restriction, cell aggregates are hindered from escaping the at least one recess of the present invention and reside in said recess. The height of the at least one chamber can be adapted to the width of the at least one recess. According to another preferred embodiment of the present invention the ratio of the distance between the base and the top of the at least one chamber to the width of the at least one recess is between 0.5 to 1 and 0.01 to 1, preferably between 0.2 to 1 and 0.02 to 1, preferably between 0.1 to 1 and 0.04 to 1.

According to another preferred embodiment of the present invention the substrate comprises, consists of or is coated with a biocompatible material.

According to another preferred embodiment of the present invention the biocompatible material is a silicone or a plastic material or glass. Silicone and glass are usually processed with standard photolithography methods and offers fast and easy fabrication of microfluidic devices. Glass is optically transparent, electrically insulating, and chemically inert. Silicon is optically transparent, oxygen permeable, cheap, and resistant to organic solvents. Plastic-based devices are also of great importance for industrial applications. The vast variety of plastic materials offers great flexibility in choosing suitable materials with specific properties. Compared with inorganic materials, polymers are easy to access, inexpensive, and therefore have become a commonly used material for microfluidic devices.

According to another preferred embodiment of the present invention the biocompatible material is selected from the group consisting of a polystyrene, cycloolefin-copolymer, polymethylmethacrylate, cyclo-olefin polymer, polydimethylsiloxane, polycarbonate (PC), polypropylene (PP), polyvinylchloride (PVC), perflouropolyether (PFPE), polyurethane, poly(ethyleneterephthalate) (PET), polyester and thiol-enes. The microfluidic device of the present invention can be produced by microfabrication methods known in the art, such as casting, 3D printing, lithography, hot embossing, or micro-injection molding.

According to another preferred embodiment of the present invention at least one recess and/or the substrate is/are coated at least partially with an anti-adhesive layer. The anti-fouling layer prevents the adhesion of cells and proteins on a surface of the at least one recess. In another preferred embodiment of the microfluidic device according to the present invention, the at least one chamber is also at least partially coated with an anti-fouling layer.

According to another preferred embodiment of the present invention the anti-adhesive layer is selected from a material of the group consisting of polyethylene glycol (PEG)-based polymers, preferably PLL-g-PEG or PEGS having different lengths, varying from about 22 to 450 repeating units, and/or corresponding to a molecular weight in the range of 1000 to 20000 Da, polybetaines, such as poly(sulfobetaine) (PSB) or poly (carboxybetaine) (PCB), polyampholytes, fluorinated-polymers, polysaccharides, such as agar or agarose, polyhydroxy- polymers, such as poly(2-hydroxyethyl methacrylate (poly-HEMA) or poly-hydroxypropyl methacrylate (poly-HPMA), poly(ethylene oxide), hydroxypropyl methylcellulose (HPMC), poly(vinyl alcohol) (PVA), poly(2-hydroxyethyl methacrylate) (pHEMA), poly(acrylic acid) (PAA), dextran, hydroxylethylcellulose (HEC), natural biopolymers (Hydrophobines, S-layer protein SbpA), anti-fouling nanointerfaces such as polyelectrolyte multilayers (PEMs), self-assembled monolayers (SAMs), nonionic surfactants such as polyoxyethylene dodecanol, Tween-20, n-dodecyl-D-maltoside (DDM) and pluronics (triblock copolymers PEO-b-poly(propylene oxide)-b-PEO), and silanes such as 3-glycidoxypropyltrimethoxysilane (GPTMS) and 3-chloropropyltrichlorosilane (CPTMS) and (3-aminopropyl)triethoxy silane (APTES).

According to another preferred embodiment of the present invention the at least one recess has the shape of an elliptic paraboloid or a hemisphere.

According to another preferred embodiment of the present invention the at least one recess having the shape of an elliptic paraboloid at least a part of a shell surface of the paraboloid is formed by a parable with the formula y=A*x^{B} rotated in space, wherein A is between 1 and 10, preferably 1, 2, 3, 4, 5, 6, 7, 8, 9 or 10, and B is 2, 4 or 6. With the at least on recess having the shape of an elliptic paraboloid or a hemisphere according to the configuration explained above a single round cell aggregate can form in the at least one recess.

According to another preferred embodiment of the present invention the at least one recess has a width of 100 um to 3 mm, preferably 200 um to 2 mm, more preferably 500 um to 1.5 mm.

According to another preferred embodiment of the present invention the base of the at least one chamber further comprises a hollow body, wherein the hollow body is fluidically connected with the at least one recess and the interior of the at least one chamber, and wherein the width of the base area of the hollow body is at least partially the width of the at least one recess. The hollow body of the present invention has the advantage that the supply of the cell aggregates with fluids in the at least one recess can be increased. It turned out that more fluid can be collected in the least one recess and that cell aggregates can be continuously supplied by liquid by introducing a further hollow body that is situated between the at least one recess and the interior of the at least one chamber. The at least one chamber, the at least one hollow body, and the at least one recess are fluidically connected, so that fluid can flow through the chamber and fill the hollow body and the recess where the cell aggregates reside. The fluid supply of the cell aggregates in the at least one recess can be modified by the shape and/or the width of the hollow body.

The "base area of the hollow body", as used herein, refers to the two-dimensional intersection area of the three-dimensional hollow body that is in contact with the base area of the at least one recess and situated on the opposite of the base of the at least one chamber. Preferably, the base area of the hollow body has the shape of a circle or an ellipse. The width of the base area of the hollow body is at least partially, preferably equal to the width of the at least one recess.

The "top area of the hollow body", as used herein, refers to the two-dimensional intersection area of the three-dimensional hollow body that is in contact with the base of the at least one chamber and situated on the opposite of the base area of the at least one recess.

According to another preferred embodiment of the present invention the width of the top area of the hollow body is between 500 µm to 3 mm, preferably between 1 mm to 2 mm, wherein the width of the hollow body is the smallest line segment between two opposite points passing through the center on the top area of the at least one recess. The width of the hollow body can be adapted to enhance fluid flow to the at least one recess.

According to another preferred embodiment of the present invention the hollow body has the shape of truncated cone, and wherein the base and the top areas of the truncated cone have the shape of a circle or an ellipse.

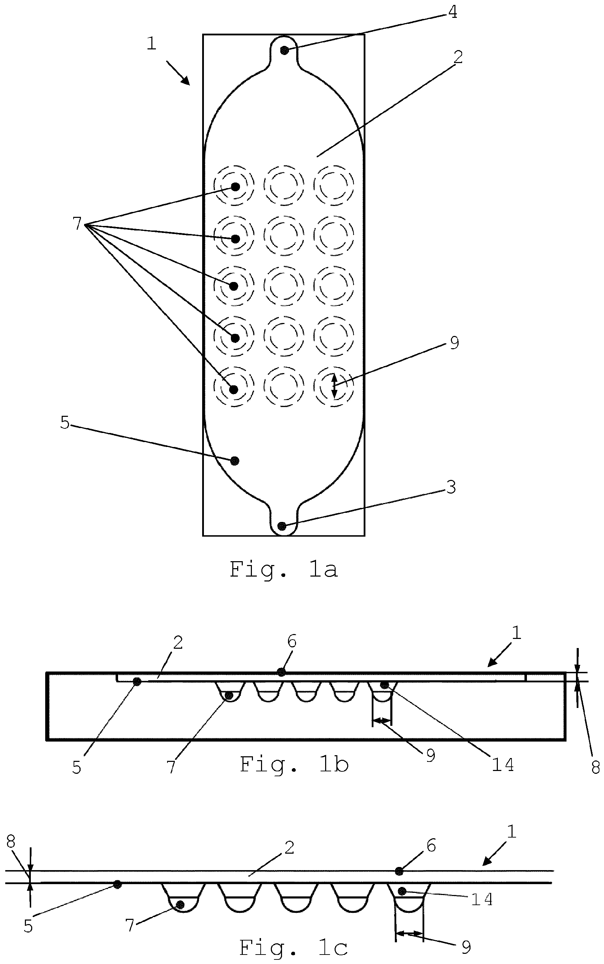

Figs. 1a to 1c show schematic plan views of an embodiment of a microfluidic device 1 according to the present invention. The microfluidic device 1 comprises a chamber 2 having a fluid inlet 3 for introducing fluid into the chamber 2 and a fluid outlet 4 for removing said fluid from the chamber 2. Moreover, the chamber 2 comprises a base 5 formed by a substrate which comprises fifteen recesses 7. The top 6 is situated on the opposite to the base 5, wherein the distance 8 between the base 5 and the top 6 is less than the width 9 of the at least one recess 7. The hollow body 14 is situated between the chamber 2 and the at least one recess 7, wherein the width of the hollow body 14 is equal to the width 9 of the at least one recess 7.

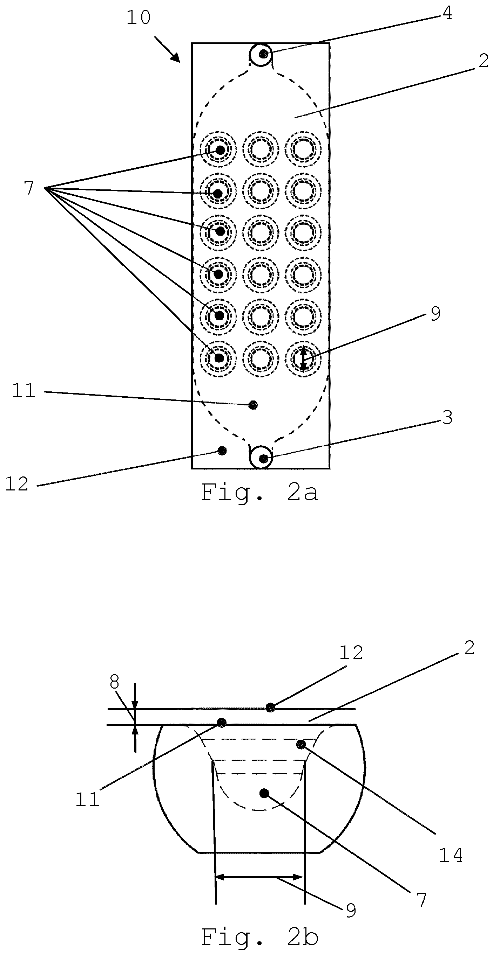

Figs. 2a and 2b show in schematic plan views of another embodiment of a microfluidic device 10 according to the present invention. The microfluidic device 10 distinguishes from the microfluidic device 1 according to Figs. 1a to 1c in the fact that a chamber 2 of the microfluidic device 1 is essentially embedded in the substrate that forms the base 5 of the chamber 2. In contrast, the microfluidic device 10 according to the present invention has a plain base 11 comprising fifteen recesses 7, wherein the chamber 2 is embedded in the top layer of the device 10. Figs. 2a and 2b show the microfluidic device 10 according to the invention with the base 11 attached to the top layer that forms the top 12 of the chamber 2. The hollow body 14 is situated between the chamber 2 and the at least one recess 7, wherein the width of the hollow body 14 is equal the width 9 of the at least one recess 7. Further, the microfluidic device 10 distinguishes to the microfluidic device 1 according to Figs. 1a to 1c in the fact, that the recesses 7 of the microfluidic device 10 are arranged in a 1536-well plate footprint. Therefore, the microfluidic device 10 of the present invention is suitable for standardized laboratory processes such as plate reader measurements, automated liquid handling procedures, automated imaging, etc.

Elements of the microfluidic device 10 being similar to elements of the microfluidic device 1 according to Figs. 1a to 1c are indicated with the same reference numerals. The base 5;11 can also comprise a different number of recesses 7.

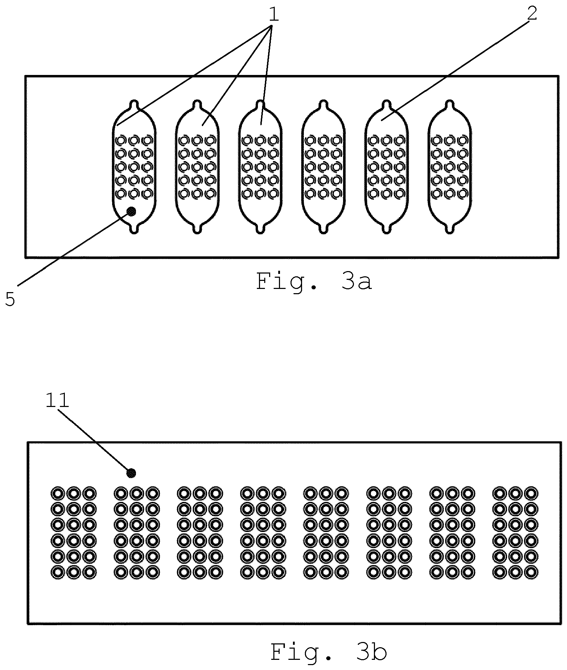

Fig. 3a shows a schematic plan view of an embodiment comprising six microfluidic devices 1 according to Figs. 1a to 1c. Individual chambers 2 of the microfluidic devices 1 are embedded in one base body.

Fig. 3b shows a schematic plan view of an embodiment comprising six microfluidic devices 10 according to Figs. 2a and 2b. Fifteen recess 7 arranged in a 1536-well plate footprint are contained in one base body.

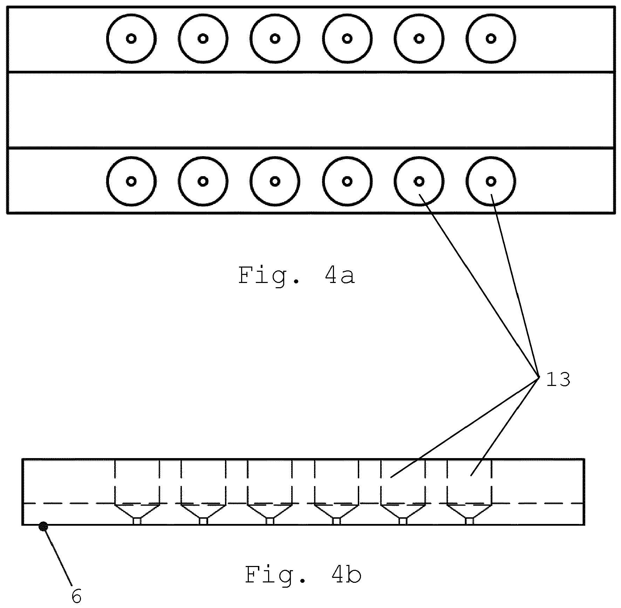

Fig. 4a and 4b show a schematic plan view of the top layer of the microfluidic device 1 according to Figs. 1a to 1c comprising six pairs of fluid reservoirs 13. The fluid reservoirs 13 are connected to one opening 3 for introducing the fluid into the chamber 2 and one opening 4 for removing the fluid from the chamber 2. The top layer can also comprise various chamber structures that fits to a plain base 11, as for example, shown an embodiment of the microfluidic device 10 according to Figs. 2a and 2b.

According to another preferred embodiment of the present invention a base body comprises the base and a top body comprises the top of the at least one chamber, and wherein the base body and the top body are reversibly sealed with each other. "Reversibly sealed", as used herein, means that the base body and the top body of the present invention can be attached and removed from each other for at least one time. After attaching the base body and the top body, the obtained microfluidic device may be leakage-free. Attachment of the base body and the top body can be done by mechanical, physical and/or chemical methods. For instance, the base body and the top body can be attached mechanically by a locking mechanism. Preferably, the base body and the top body are attached to each other by an adhesive. The base body and the top body of the present invention can be removed from each other by simply opening the locking mechanism or by lifting off the top body from the base body so that both bodies may be indestructibly released from the adhesive. After the removal of the top body from the base body, the open side of the at least one recess of the present invention is accessible.

Another aspect of the present invention relates to a method for the storage and/or transport of cell aggregates. A fluid comprising cells is applied to the at least one chamber of the device as defined above, and the cells are incubated until at least one aggregate is formed in the at least one recess. According to the present invention the cell aggregates can be stored and/or transported for a period between 1 day to 10 days, preferably between 2 to 8 days.

In particular, the present invention provides a method for the storage and/or transport of cells, preferably eukaryotic cells, preferably mammalian cells, preferably stem cells and/or tumor cells.

Surprisingly, it turned out that extracellular vesicles can also be stored and/or transported by the method of the present invention. Extracellular vesicles can have a diameter of 20 to 5000 nm and can be secreted by a wide variety of cell types. In general, extracellular vesicles such as exosomes, microvesicles, and apoptotic bodies are membrane-bound and can be loaded, for instance, with a therapeutic cargo. Exosomes are a type of extracellular vesicle that can be secreted by most eukaryotic cells. Microvesicles are another type of extracellular vesicles that are outward budded from cell surface membrane. Apoptotic bodies, on the other hand, are extracellular vesicles that are formed from dead cell debris. Exosomes, microvesicles, and apoptotic bodies can be released in vivo or in vitro, such as in cell culture. It is known that extracellular vesicles secreted from various cells function as an intermediary of intercellular communication in vivo and play a significant role in physiological phenomena of multiple diseases such as cancer.

Preferably, the cells are stored and/or transported in the form of aggregates. It turned out that cells can be transported and/or stored in a compact and cost-effective manner in the form of aggregates by the method of the present invention. Thus, the shipping process is simple and efficient with low cost due to the decreased size of the required packaging. The method of the present invention requires much less reagents than other live cell shipping techniques.

The term "cellular viability", as used herein, refers to the number of living eukaryotic and/or prokaryotic cells in a cell population.

Surprisingly, the method of the present invention shows to be a reliable and cost-efficient method for the transport and/or storage of cell aggregates while ensuring good cellular viability and a natural cell environment through three-dimensional cell cultivation throughout storage and/or transport, preferably of eukaryotic and/or prokaryotic cells. Preferably, the fluid comprising the cells also contains nutrients to supply the cells, such as cell culture media. In order to use extracellular vesicles for analysis and diagnosis, it is first necessary to concentrate and recover the vesicles from biological samples such as blood, urine, or saliva. The concentrated extracellular vesicles can be easily and safely stored and/or transported by the method of the present invention.

Another advantage of the present invention relates to the possibility to store and/or transport cells of different types, sources and/or patients. The method of the present invention has the advantage that a single device is capable of storing and/or transporting different samples without the need for an increased package size. Different samples in the form of cells, in particular of cell aggregates can be stored and/or transported in different chambers of the device, allowing a direct classification of each cell type, patient, etc. to each chamber. The method of the present invention requires much less reagents than other live cell shipping techniques. According to the method of the present invention at least one device, preferably at least two devices, preferably at least three devices, preferably at least four devices, preferably at least five devices, preferably at least ten devices, preferably at least twenty devices, preferably at least thirty devices, preferably at least forty devices, preferably at least fifty devices, preferably at least sixty devices, preferably at least seventy devices, preferably at least eighty devices, preferably at least ninety devices, preferably at least one hundred devices can be stored/ and or transported simultaneously.

According to another preferred embodiment of the present invention the microfluidic device is centrifuged after step a) at a speed of 50 to 500 rcf, preferably at 150 to 300 rcf to promote the aggregation of the cells. It turned out, that the aggregation time and the shape of the aggregates of the cells can be efficiently optimized and enhanced by centrifugation of the device.

In order to mimick the natural environment of the cell aggregate, preferably the cell aggregate within the microfluidic device of the present invention, it is particularly preferred to apply a hydrogel to the at least one chamber of the microfluidic device. For instance, the hydrogel can form a matrix for the spheroid to be produced.

According to another preferred embodiment of the present invention a hydrogel is introduced into the at least one chamber after step a) or b), wherein the hydrogel consists of at least one compound selected from the group consisting of polyethylene glycol, polyacrylamide, dextran, collagen, fibrin, fibronectin, laminin, hyaluronic acid, fibroin, alginate, cellulose or combinations thereof.

It is preferred, that the cell aggregates can be harvested after storage and/or transport from the microfluidic device according to the invention. After harvesting, cell aggregates can further be used for e.g., biomedical research, pharmaceutical production, personalized medicine approaches etc. To harvest the cell aggregates from the device, the hydrogel may have to be digested to access the respective cell aggregates. According to another preferred embodiment of the present invention the hydrogel is digested by an enzyme selected from the group consisting of collagenase, hyaluronidase, cellulase, ethylenediaminetetraacetic acid (EDTA), sodium citrate, dispase, alpha-chymotrypsin, N-acetyl-cysteine, glutathione, dithiothreitol and combinations thereof.

According to another preferred embodiment of the present invention the at least one aggregate and/or the cells is/are collected from the device.

According to another preferred embodiment of the present invention the at least one cell aggregate and/or cell are collected by separating the top body and the bottom body as mentioned above by collecting the at least one cell aggregate and/or cell from the at least one recess. In particular, the top body and the bottom body of the device of the present invention that is reversibly sealed, are removed from each other and the cell aggregates are collected by e.g., pipetting from the device. Alternatively, the hydrogel can also be digested by an enzyme after separating the top body and the bottom body. In this case, top body and the bottom body can be carefully removed, and the enzyme can be added to the hydrogel comprising the cell aggregates. After digestion of the hydrogel, the cell aggregates can be collected from the device.

The cell aggregates can also be disaggregated into individual cells. In particular, spheroids or organoids can be disaggregated into individual cells or aggregates consisting of extracellular vesicles can be disaggregated into individual extracellular vesicles. This has the advantage that, e.g. in the case of eukaryotic cells, individual cells can be further passaged as a monolayer culture in cell culture flasks. According to another preferred embodiment of the present invention the cells are collected by disaggregating the at least one aggregate into cells, and wherein said cells are collected through the at least one opening and/or by collecting said cells from the at least one chamber by separating the top body and the bottom body, as defined above.

According to another preferred embodiment of the present invention the disaggregation is achieved by applying a fluid comprising trypsin, collagenase, accutase, dispase, elastase, ethylenediaminetetraacetic acid (EDTA), sodium citrate or combinations thereof to the at least one aggregate.

One further aspect of the present invention relates to the use of the device as defined above for the storage and/or transport of cells and/or cell aggregates. In particular, the microfluidic device of the present invention can be used for the shipment of viable cells in a compact, reliable, and cost-efficient manner. In addition, the device can be used for personalized medicine approaches. For instance, eukaryotic cells, preferably tumor cells or extracellular vesicles from blood or urine of a patient can be collected and shipped by using the device of the present invention to a required destination for screening, evaluation, and selection of proper treatment. One major advantage of the present invention relates to the small amounts of patient samples needed compared to standard shipment options.

Another aspect of the present invention relates to the method for the screening of compounds on cell aggregates. In particular, the method of the present invention can be used for the screening of chemical libraries of synthetic molecules, natural products or extracts, traditional small-molecule drugs, usually derived from chemical synthesis, and bio-pharmaceuticals, which include recombinant proteins, vaccines, blood products used therapeutically (such as intravenous immunoglobulins), gene therapy, monoclonal antibodies and cell therapy (for instance, stem-cell therapies). The application of varying concentrations of a compound on cell aggregates allows the determination of whether a substance has a beneficial or negative effect on cells within a cellular spheroid. Such a method can be used, for instance, to identify doses of compounds that may have beneficial, toxic, inhibitory and/or stimulating effects on cell aggregates.

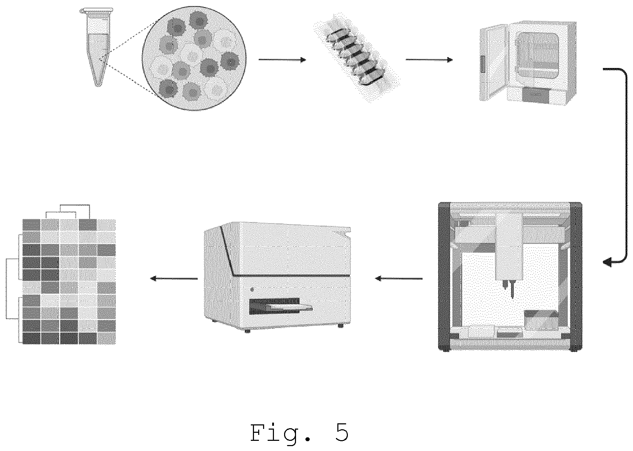

Fig. 5 shows a schematic workflow of the method of an embodiment of the present invention, including the application of a cell suspension to the device of the present invention, incubation of the device to form cell aggregates, treatment of the cell aggregates with at least one compound by applying the compound by a pipetting robot and measuring the effects of the compound on the aggregates by high-content and/or high-throughput screening.

Preferably, the cells are obtained from a mammalian source, preferably from a human. With the method of the present invention, physiological parameters and/or the state of a disease can be determined "from bench-to-bedside", meaning that the results obtained with the method of the present invention can be directly used to develop a treatment regimen for patients.

According to another preferred embodiment of the present invention the cells are tumor cells. Preferably, the tumor cells are selected from the group consisting of benign adenoma tumor cells, angiofibroma tumor cells, hemangioma tumor cells, leiomyoma tumor cells (fibroid), benign chorangioma tumor cells, benign colonic tumor cells, cystadenoma tumor cells, dermoid tumor cells, desmoid tumor cells, ductal carcinoma in situ (DCIS) tumor cells, fibroadenoma tumor cells, fibroma tumor cells, benign ganglioneuroma tumor cells, lipoma tumor cells, meningioma tumor cells, myxoma tumor cells, neurofibroma tumor cells, nevus tumor cells, osteochondroma tumor cells, pheochromocytoma tumor cells, polyposis tumor cells, schwannoma tumor cells, prostatic intraepithelial neoplasia tumor cells, benign prostatic hyperplastic (hypertrophic) tumor cells, benign teratoma tumor cells, benign thymoma tumor cells and Brenner tumor cells.

According to another preferred embodiment of the present invention the tumor cells are obtained by dissociating a solid tumor. A "solid tumor", as used herein, refers to an abnormal mass of tissue that usually does not contain cysts or liquid areas. Solid tumors may be benign (not cancer), or malignant (cancer). Different types of solid tumors are named for the type of cells that form them. Examples of solid tumors are sarcomas, carcinomas, and lymphomas.

### EXAMPLE

### Materials and Methods

### Fabrication of the microfluidic device:

The microfluidic device according to the present invention was fabricated by double-casting of polydimethyl siloxane (PDMS). The master mold, including recess- and chamber structures, was manufactured in polymethyl methacrylate (PMMA) by CNC micromilling (Denz-Biomedical, Austria). PDMS (Sylgard 184 Silicon Elastomer, Farnell, Austria) was mixed with the curing agent in a weight ratio of 10:1. The polymer was degassed in a vacuum chamber for 1 h, poured onto the PMMA structure, and baked for 2 h at 80 °C. The structure was peeled off from the PMMA matrix subsequently and was hard baked for 48 h at 90 °C. As a result, the final PDMS mold for the base layer fabrication was obtained. To remove PDMS structures from molds properly, the surface of the PDMS mold was plasma-activated and silanized with trichloro(1H,1H,2H,2H-perfluorooctyl) silane (Sigma-Aldrich, Austria) for 10 min under vacuum and baked for 1 h at 80 °C. Molds for top layer, including reservoirs, were 3D printed by iMaterialise (Denmark). The PDMS master mix was poured into 3D printed molds and baked for 2 h at 70 °C. Before bonding, each chamber was coated with 0.5% wt antifouling Lipidure-CM5206 solution (AMSbio, UK) for 1 h at 80 °C. Holes of 1.5 mm diameter were punched through the reservoir layer with biopsy punchers to connect the reservoirs and the chambers. The two PDMS layers (base layer and top layer with reservoirs) were bonded by O₂-plasma activation for 30 s, 0.9 mbar, 200 W (Diener, Germany) and baked at 80 °C overnight.

### Cell culture:

HeLa cells were expanded in T75 Flasks (Greiner, Austria) containing 10 mL DMEM cell culture media (Sigma-Aldrich, Austia) supplemented with 10% FBS (Sigma-Aldrich, Austria, 2 mM L-glutamine (Sigma-Aldrich, Austria) and 1% penicillin/streptomycin and cultivated at 37°C with 5% CO₂. The cells were expanded to 80-90% confluency for seeding into the microfluidic device. Prior to seeding the cells are detached from the expansion flask by washing once with PBS (Sigma-Aldrich, Austria) and treated with 2 mL trypsin-EDTA solution (Sigma-Aldrich, Austria) for 5 minutes. The detached cells were collected, counted and the concentration adjusted accordingly in complete growth media.

### Cell loading:

Before cell seeding, the chambers were filled with 70% ethanol and placed in an ultrasonic bath to remove air bubbles. The device was sterilized by washing 3× with 200 µL of 70% ethanol and three times with 200 µL of 1× PBS (Sigma-Aldrich, Austria) supplemented with 1% of penicillin/streptomycin (Sigma-Aldrich, Austria) to clear the chambers from ethanol. The devices were maintained and incubated in quadriPERM chambers (Sarstedt, Austria) filled with 2 mL 1× PBS supplemented with 1% antibiotic/antimycotic solution (Sigma-Aldrich, Austria) to avoid liquid evaporation. Prior to cell seeding, PBS was removed from all reservoirs and preconditioned with 200 µL cell culture medium. After removal of medium, 100 µL of a cell suspension was added to each chamber. On the next day, chambers were flushed with 200 µL growth medium to remove excess cells.

### Evaluation of cellular viability:

Cell viability was determined by live dead staining. Therefore, 10 pg/mL Hoechst 33342 (Sigma-Aldrich, Austria), 2 µM Calcein-AM (Invitrogen, Austria) and 4 µM EtBr (Invitrogen, Austria) were mixed in complete growth medium and applied to the cell aggregates in the microfluidic device. The microfluidic devices were placed in a fluorescent microscope (Olympus IX83) at 37°C and 5% CO₂ and images were taken after a 30 h incubation period. The fluorescent intensity of the different channels (blue/Hoechst 33342/total cell count; green/Calcein-AM/viable cells; red/EtBr/dead cells) of the cell aggregates was measured with ImageJ.

### CFD simulation:

All designs for the CFD simulation were made with AutoCAD software (Autodesk). SAT files from the generated designs were loaded into Autodesk CFD software (Autodesk). The properties of water (Autodesk pre-set) were assigned to the model while the simulated flow rate was set to 10 µL/min and atmospheric pressure was set for the outlet part of the microfluidic device. All models were simulated with these settings.

### Results

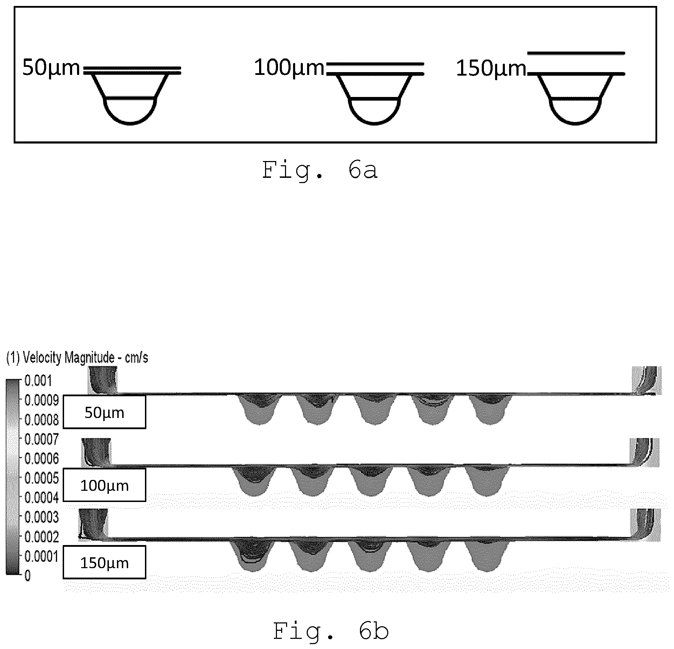

To estimate the flow profiles of the microfluidic flow in the device according to the present invention under different operating conditions, computational fluid dynamics (CFD) simulations were performed. Velocity magnitudes were determined in silico to assess the impact of three different channel heights at a fixed simulated flow rate of 10 pL/min. The results in Figs. 6a and 6b show a homogenous distribution of the fluid flow at lower chamber heights of 50 µm compared to higher heights of 150 µm. These findings surprisingly show that lower chamber heights result in fluid streamlines that uniformly envelop the entire array of recesses of the microfluidic device without turbulences compared to higher chamber heights, thus pointing at a more efficient medium turnover inside the recesses at lower chamber heights.

In addition, it has been observed that loss and/or damage of spheroids can be avoided by the microfluidic device having lower a chamber height than the width of the recesses compared to a device having a chamber height larger than the width of the recesses.

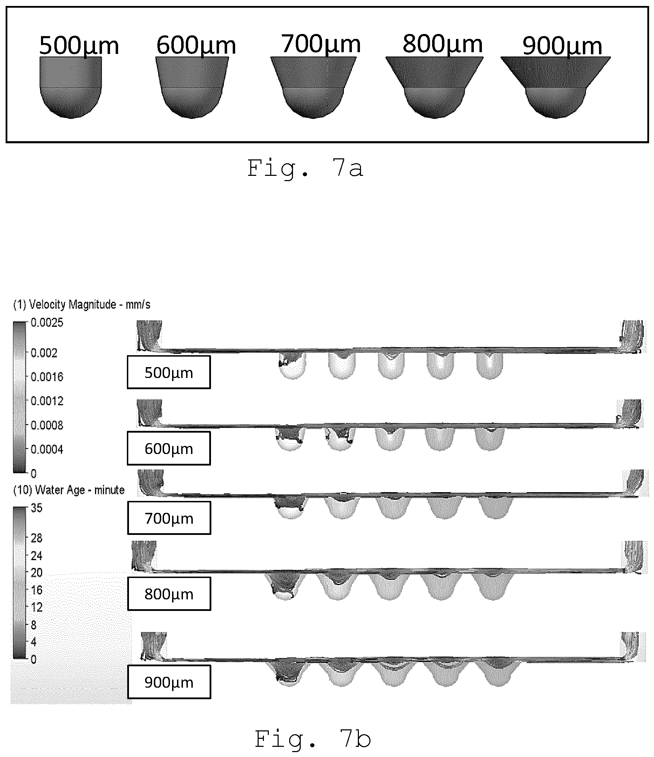

To assess the influence of the hollow body width on flow profiles of the microfluidic flow in the device, (CFD) simulations at a constant simulated flow rate of 10 pL/min were performed. The results in Figs. 7a and 7b exhibit that fluid flow in the recesses can be enhanced by increasing the widths of the hollow body. This effect leads to a better fluid supply of, e.g. cells, by nutrients and improved trapping of cells in a recess during applying a cell suspension.

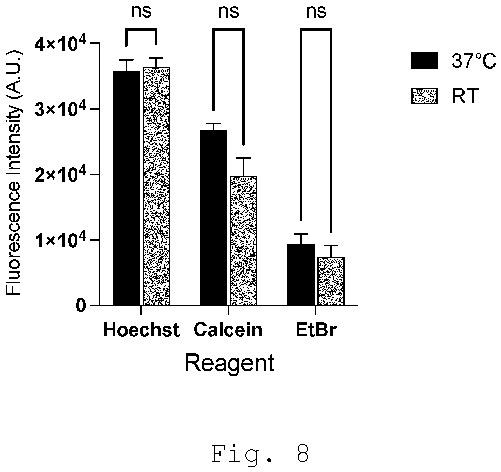

To evaluate optimal storge and/or storage conditions of cells in the microfluidic device according to the invention, HeLa cells were applied to two different devices: One device was stored at cell culture conditions (at 37°C, 5% CO₂ atmosphere) and the second device was stored at room temperature. After storage of both devices overnight, the viability of the cells in the form of spheroids was assessed. The results in Fig. 8 show that he cultured cell aggregates did not lose significant viability after cultivation in the microfluidic device after 24 h when comparing with 37°C, 5% CO₂ and room temperature cultivation conditions.

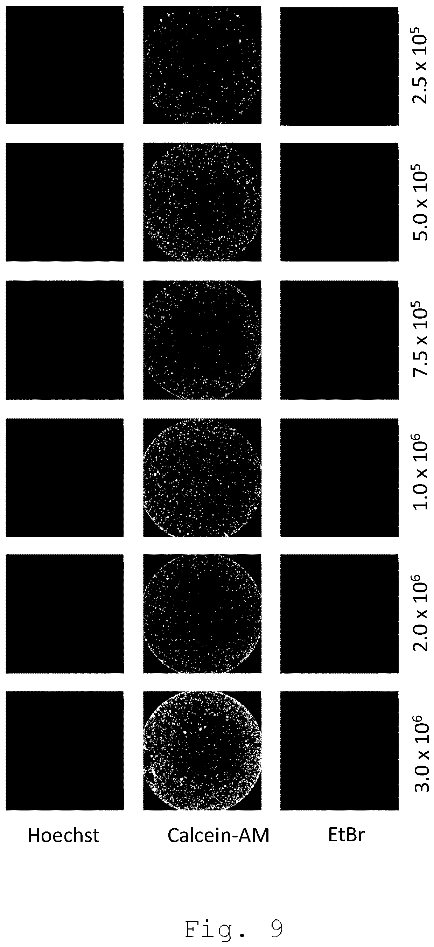

Additionally, cell aggregates cultured at room temperature for 48 h could be harvested from the microfluidic device through digestion of the aggregates and removal of the liquid containing cells. Furthermore, the harvested cell suspensions revealed the same trend in cell concentration when seeded in a cell culture plate as was seeded in the microfluidic device, as shown in Fig. 9. These results demonstrate that viable cells over a broad range of seeding densities were obtained in culture flasks after storage in the form of cell aggregates in the microfluidic device of the present invention.

### Conclusion:

The design of the microfluidic device was optimized for a streamline (e.g., no voids and dead volumes) flow profile through adaptation of channel height and recess shapes. The results from CFD simulations determined that a decrease in channel height have a major impact on the flow profile in the microfluidic device. This optimized flow profile results in a better nutrient supply of the entrapped cell aggregates while it simultaneously improved the trapping of cells in the recesses. The microfluidic device is able to form cell aggregates from a solution containing individual cells and retain similar cell number in each recess. In summary, the microfluidic device according to the present invention is a facile and cost-effective tool for storage and/or transport of cell aggregates for a broad range of applications.

## Claims

1. A microfluidic device (1;10) for the storage and/or transport of cells, in particular cell aggregates, wherein the device (1;10) comprises at least one chamber (2) comprising at least one opening (3) for introducing a fluid into the chamber (2) and at least one opening (4) for removing a fluid from the chamber (2), wherein the at least one chamber (2) comprises a base (5;11) and a top (6;12), wherein the top (6;12) is situated on the opposite to the base (5;11), wherein the base (5;11) is formed by a substrate comprising at least one recess (7) for collecting a fluid, wherein the distance (8) between the base (5;11) and the top (6;12) of the at least one chamber (2) is less than the width (9) of the at least one recess (7), and wherein the width (9) of the at least one recess (7) is the smallest line segment between two opposite points passing through the center of the base area of the at least one recess (7) .

2. The device (1;10) according to claim 1, wherein the at least one opening (3,4) is connected to at least one fluid reservoir (13).

3. The device (1;10) according to claim 1 or 2, wherein the ratio of the distance (8) between the base (5;11) and the top (6;12) of the at least one chamber (2) to the width (9) of the at least one recess (7) is between 0.5 to 1 and 0.01 to 1, preferably between 0.2 to 1 and 0.02 to 1, preferably between 0.1 to 1 and 0.04 to 1.

4. The device (1;10) according to any one of claims 1 to 3, wherein the at least one recess (7) has the shape of an elliptic paraboloid or a hemisphere.

5. The device (1;10) according to any one of claims 1 to 4, wherein the at least one recess (7) has a width (9) of 100 um to 3 mm, preferably 200 um to 2 mm, more preferably 500 um to 1.5 mm.

6. The device (1;10) according to any one of claims 1 to 5, wherein the base (5;11) of the at least one chamber (2) further comprises a hollow body (14), wherein the hollow body (14) is fluidically connected with the at least one recess (7) and the interior of the at least one chamber (2), and wherein the width of the base area of the hollow body (14) is at least partially the width (9) of the at least one recess (7).

7. The device (1;10) according to claim 6, wherein the width of the top area of the hollow body (14) is between 500 µm to 3 mm, preferably between 1 mm to 2 mm, wherein the width of the hollow body (14) is the smallest line segment between two opposite points passing through the center on the top area of the at least one recess (7).

8. The device (1;10) according to claim 6 or 7, wherein the hollow body (14) has the shape of truncated cone, and wherein the base and the top areas of the truncated cone have the shape of a circle or an ellipse.

9. The device (1;10) according to any one of claims 1 to 8, wherein a base body comprises the base (5;11) and a top body comprises the top (6;12) of the at least one chamber (2), and wherein the base body and the top body are reversibly sealed with each other.

10. A method for the storage and/or transport of cells, in particular cell aggregates, comprising the steps of:
a) applying a fluid comprising cells into the at least one chamber (2) of the device (1;10) according to any one of claims 1 to 9 and thus providing said fluid to the at least one recess (7), and
b) incubating the cells until at least one aggregate is formed in the at least one recess (7).

11. The method according to claim 10, wherein a hydrogel is introduced into the at least chamber (2) after step a) or b), wherein the hydrogel consists of at least one compound selected from the group consisting of polyethylene glycol, polyacrylamide, dextran, collagen, fibrin, fibronectin, laminin, hyaluronic acid, fibroin, alginate, cellulose or combinations thereof.

12. The method according to claim 11, wherein the hydrogel is digested by an enzyme selected from the group consisting of collagenase, hyaluronidase, cellulase, ethylenediaminetetraacetic acid (EDTA), sodium citrate, dispase, alpha-chymotrypsin, N-acetyl-cysteine, glutathione, dithiothreitol and combinations thereof.

13. The method according to any one of claims 10 to 12, wherein the at least one cell aggregate and/or the cells is/are collected from the device (1;10) after step b).

14. Use of the device (1;10) according to any one of claims 1 to 9 for the storage and/or transport of cells, in particular cell aggregates.

15. A method for the screening of an effect of compounds on cell aggregates comprising the steps of:
a) providing a solution comprising cells,
b) applying the solution of step a) into the at least one chamber (2) of the device (1;10) according to any one of claims 1 to 9 and thus providing said fluid to the at least one recess (7),
c) incubating the cells until at least one cell aggregate is formed in the at least one recess (7),
d) treating at least one cell aggregate with at least one compound, and
d) measuring an effect of the at least one compound on said at least one cell aggregate.
